## Europäisches Patentamt
## European Patent Office
## Office européen des brevets

(19)

(11) Veröffentlichungsnummer: **0 184 746**
**B1**

(12) **EUROPÄISCHE PATENTSCHRIFT**

(45) Veröffentlichungstag der Patentschrift:
**22.03.89**

(51) Int. Cl.⁴: **A 61 C 19/04**, G 01 N 3/30

(21) Anmeldenummer: **85115257.9**

(22) Anmeldetag: **02.12.85**

(54) **Zahnärztliches Perkussionsinstrument.**

(30) Priorität: **13.12.84 DE 3445529**

(43) Veröffentlichungstag der Anmeldung:
**18.06.86 Patentblatt 86/25**

(45) Bekanntmachung des Hinweises auf die Patenterteilung:
**22.03.89 Patentblatt 89/12**

(84) Benannte Vertragsstaaten:
**CH DE FR IT LI SE**

(56) Entgegenhaltungen:
**EP-A- 0 093 895**

(73) Patentinhaber: **Siemens Aktiengesellschaft Berlin und München, Wittelsbacherplatz 2, D-8000 München 2 (DE)**

(72) Erfinder: **Wohlgemuth, Jürgen, Brüder-Knaus-Strasse 61, D-6100 Darmstadt (DE)**

## Beschreibung

Die Erfindung bezieht sich auf ein zahnärztliches Perkussionsinstrument mit einem als Handstück ausgebildeten Instrumentengehäuse, in dem ein an seinem einen Ende einen Prüfkopf und an seinem anderen Ende einen Permanentmagneten enthaltender Stössel axial beweglich gelagert ist sowie mit einer im Instrumentengehäuse fest angeordneten Magnetspule, die zusammen mit dem Permanentmagneten den Antrieb für den Stössel bildet.

Bei einem bekannten solchen Perkussionsinstrument (EP-A-93 885) besteht der Antrieb für die Vor- und Rückwärtsbewegung des Stössels aus einer den Stössel über einen grossen Teil seiner Länge konzentrisch umgebenden Antriebsspule und einer an dem den Prüfkopf abgewandten Ende des Stössels angeordneten Dauermagnetkörper. Der Dauermagnetkörper, der im Vergleich zum Stössel einen sehr viel grösseren Aussendurchmesser hat und auch dazu dient, zusammen mit einer ebenfalls konzentrisch dazu im Gehäuse angeordneten Messspule eine Messgrösse zur Bestimmung der Stösselgeschwindigkeit zu liefern, trägt stirnseitig noch ein Flussleitstück sowie einen Beschleunigungsaufnehmer.

Sowohl die den Stössel konzentrisch umgebende Magnetspule für den Antrieb als auch der am Stösselende angeordnete Dauermagnetkörper mit Flussleitstück und Beschleunigungsaufnehmer führen dazu, dass das Handstück in seinen Aussenabmessungen relativ gross und damit unhandlich wird.

Bei einem anderen bekannten Perkussionsinstrument (DE-A-26 17 779) ist konzentrisch zum Stössel eine Druckfeder angeordnet. Auch bei diesem Instrument ist der Aussendurchmesser relativ gross.

Um der Forderung, auch an relativ schlecht zugänglichen Stellen im Patientenmund mit dem Instrument eine sichere Diagnose durchführen zu können, gerecht zu werden, ist anzustreben, das Instrument gerade im vorderen Bereich, in dem sich bei den bekannten Instrumenten der Antrieb befindet, möglichst schlank, also im Durchmesser so klein wie möglich, zu halten.

Ein weiteres Problem liegt darin, den Stössel nach Abschalten des Antriebs bis zum Aufprall auf das Objekt mit gleichbleibender Geschwindigkeit zu bewegen. Bei den bekannten Perkussionsinstrumenten ist dies nicht mit der erwünschten Genauigkeit möglich, da dort der Stössel über seine Länge gesehen zum Teil erhebliche Querschnittsvergrösserungen aufweist, die nicht nur den Durchmesser erhöhen, sondern auch einen unerwünschten Bremseffekt zur Folge haben.

Der im Anspruch 1 angegebenen Erfindung liegt die Aufgabe zugrunde, ein Perkussionsinstrument anzugeben, mit dem sich die oben erwähnten Nachteile vermeiden lassen.

Dadurch, dass gemäss der Erfindung die Antriebsmittel hinter dem Stössel bzw. teilweise in dessen Innerem angeordnet sind und der Stössel selbst über seine Länge eine in Form und Abmessungen gleichbleibende Querschnittsfläche aufweist, lässt sich einerseits der beim Stand der Technik vorhandene unerwünschte Bremseffekt vermeiden und andererseits ein extrem kleiner Instrumentenaussendurchmesser erzielen, der nunmehr im wesentlichen nur vom Stössel, seinen Lagern und den Gehäusewandungen bestimmt ist.

Die Magnetspule bildet vorteilhafterweise zusammen mit dem rückwärtigen Teil des Instrumentengehäuses und der Versorgungsleitung eine nicht (betriebsmässig) trennbare Baueinheit, die mit dem übrigen Instrumententeil durch eine geeignete, leicht lösbare Verbindung, z.B. durch eine Schraub- oder Steckverbindung, verbunden ist. Durch eine solche Anordnung lässt sich die Anzahl der Trennstellen für die elektrischen Leitungen reduzieren, die bei den bekannten Instrumenten dieser Gattung vorhanden wären, wenn man das Instrument trennbar ausbilden würde, was aus Reinigungs-, Reparatur- oder sonstigen Gründen sinnvoll ist.

Die elektrische Verbindung der Leitungen, die zum Instrumentenvorderteil geführt werden müssen, kann vorteilhafterweise durch eine geeignete konstruktive Gestaltung eines die Antriebsspule umgebenden Rückschlussringes erfolgen, z.B. indem der Rückschlussring axial über die Antriebsspule verlängert ist und in voneinander isolierte, achsparallel verlaufende Segmente unterteilt ist, die zur Kontaktübertragung verwendet werden und mit entsprechenden federnden Gegenkontakten im anderen Handstückteil zusammenwirken. Vorteilhafterweise ist die Oberfläche des aus Weicheisenmaterial bestehenden Rückschlussringes zumindest im Bereich der Kontaktierung mit einem geeigneten Kontaktwerkstoff, wie Nikkel, Silber, Gold, Platin, Rhodium, beschichtet.

Für eine Handhabung des Instrumentes ist es günstig, wenn der mit der Versorgungsleitung vorteilhafterweise ebenfalls nicht trennbar verbundene, die Antriebsspule enthaltende Teil des Instrumentengehäuses eine Länge von maximal $1/3$ bis $1/4$ der Gesamtlänge des Instrumentes aufweist.

Der im vorderen Teil des Instrumentengehäuses angeordnete Stössel ist in Gleitlagern gelagert; diese können vorteilhafterweise eine oder mehrere axiale Durchbrechungen aufweisen, so dass benachbarte, durch Gehäuse und Stössel gebildete Kammern für einen Luftaustausch miteinander in Verbindung stehen.

Sieht man, wie gemäss einer weiteren vorteilhaften Ausgestaltung der Erfindung vorgeschlagen wird, anstelle des bei den bekannten Perkussionsinstrumenten vorgesehenen biegeschlaffen Zuleitungskabels, welches den Beschleunigungsaufnehmer mit der Messelektronik verbindet, eine Zuleitung aus elektrisch leitendem, isoliertem Draht vor, die spiralförmig konzentrisch um den Stössel herumgewickelt ist und federelastische Eigenschaften aufweist, so lässt sich damit auch bezüglich der Leitungsführung mit einem Minimum an Platzbedarf auskommen. Die Verwendung einer Spiralfeder hat darüber hinaus den Vorzug, dass ein Verdrillen bzw. Verschlingen des Kabels ausgeschlossen ist und die dabei auftretende Gefahr eines Kabelbruches vermieden wird. Durch die nunmehr definierte Anordnung der Zuleitung wird auch vermieden, dass durch Reibung des Kabels an der Gehäusewandung die Isolation beschädigt und damit das Instrument defekt wird.

Die Spiralfeder kann vorteilhafterweise aus einem Litzendraht mit zwei verdrillten Einzeldrähten oder einer Koaxialleitung bestehen. Vorteilhafterweise ist die Feder im eingespannten Zustand so weit zusammengedrückt, dass ihre Vorspannkraft der Reibungskraft entspricht und dieser bei der Vorwärtsbewegung des Stössels entgegenwirkt. Der Vorspannweg der Feder ist dabei sehr viel grösser als der Stösselhub, so dass die Federkraft über den gesamten Stösselhub annähernd konstant bleibt. Damit lassen sich die unerwünschten Reibungskräfte der Stössellager während der Vorwärtsbewegung weitgehend kompensieren.

Nachfolgend wird ein Ausführungsbeispiel der Erfindung anhand der Zeichnung näher erläutert. Es zeigen:

Figur 1 das erfindungsgemässe Perkussionsinstrument im Längsschnitt,

Figur 2 den rückwärtigen Instrumententeil im Längsschnitt,

Figur 3 einen Querschnitt entlang der Linie II–II in Figur 1,

Figur 4 einen Teil des in Figur 1 gezeigten Instrumentes im Längsschnitt,

Figur 5 eine andere Ausführungsform vom rückwärtigen Instrumententeil,

Figur 6 einen Ausschnitt von dem zum Gegenstand nach Figur 5 passenden vorderen Instrumententeil.

In einem als Handstück ausgebildeten Instrumentengehäuse 1 von etwa 150 mm Länge und 15 mm Dicke ist ein langgestreckter Stössel 2 in Lagern 3, 4 weitgehend reibungsfrei axial beweglich gelagert. Der Stössel 2 hat über seine gesamte Länge gleichbleibenden Querschnitt und enthält an seinem einen Ende einen Prüfkopf 5 und an seinem anderen Ende einen stirnseitig in einer entsprechenden Ausnehmung gefassten Permanentmagneten 7. Dem Permanentmagneten 7 korrespondierend, also axial hinter dem Permanentmagneten, ist im Gehäuse 1 eine Magnetspule 8 angeordnet. Magnetspule 8 und Permanentmagnet 7 bilden den Antrieb für die Vor- und Rückwärtsbewegung des Stössels 2.

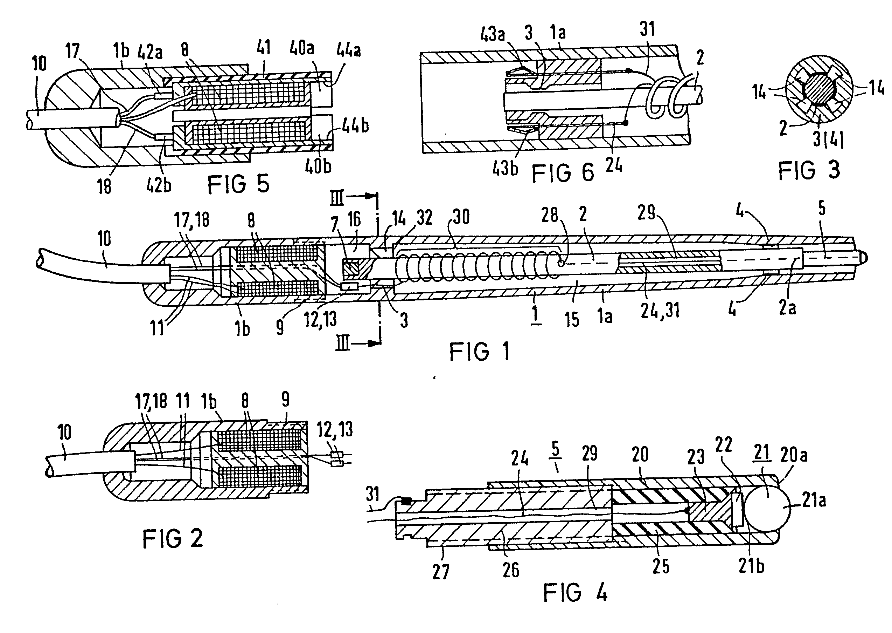

Das Instrumentengehäuse 1 ist durch eine Schraubverbindung 9 unterteilt in einen vorderen, den Stössel 2 aufnehmenden Abschnitt 1a, der den Permanentmagneten 7 enthält, und einen rückwärtigen, die Antriebsspule 8 enthaltenden Abschnitt 1b. Dieser rückwärtige Instrumentenabschnitt ist in Figur 2 als Einzelteil dargestellt. Die Antriebsspule 8 ist integraler Bestandteil des mit dem Versorgungsschlauch 10 vorteilhafterweise fest verbundenen Gehäuseteils 1b. Bei einem Wechsel des vorderen Instrumententeils 1a, z.B. zum Zwecke der Reinigung, Sterilisation oder zu anderen Zwecken, brauchen deshalb die zur Antriebsspule führenden elektrischen Leitungen 11 nicht getrennt zu werden; es ist demnach nur eine Steckverbindung 12, 13 für Leitungen 24, 31, deren Zweck später noch erläutert wird, an der Trennstelle zu lösen.

Die beiden Lager 3, 4 sind als Gleitlager ausgebildet, welche, entsprechend der Querschnittsdarstellung nach Figur 3, mehrere axiale Durchbrechungen 14 aufweisen, wodurch die zwischen Gehäuse und Stössel gebildeten Kammern 15 und 16 miteinander Verbindung haben. Bei Bewegung des Stössels können sich so Luftbewegungen zwischen den Kammern ausgleichen.

Anhand der Figur 4 wird der Aufbau des Prüfkopfes 5 näher erläutert.

Der Prüfkopf 5 wird gebildet durch eine äussere Hülse 20, an deren vorderem Ende eine als Prallkörper dienende Kugel 21 gefasst ist. An der Kugel 21 liegt ein als Beschleunigungsaufnehmer dienendes piezokeramisches Element 22 praktisch punktförmig (Position 21b) an. Das Piezoelement 22 ist mit einem Kontaktstück 23 verbunden, an das eine Signalleitung 24 angeschlossen ist. An einem Isolierteil 25 liegt ein Druckstück 26 an, welches mit einem durchgehenden Schraubgewinde 27 versehen ist, wodurch sich das Druckstück 26 einerseits in die ebenfalls mit einem Gewinde versehene, die Kugel aufnehmende Hülse 20 und andererseits in das ebenfalls mit einem Gewinde versehene Stösselende 2a einschrauben lässt.

Die Leitung 24, mit der die Beschleunigungssignale an eine extern des Instruments vorgesehene Auswerteelektronik gegeben werden, ist zusammen mit einer Masseleitung 31 bis zu einer radialen Austrittsöffnung 28 (Figur 1) im Inneren des Stössels 2 geführt, wozu dieser zumindest in diesem Bereich einen entsprechenden Kanal 29 aufweist. Danach sind die Leitungen 24, 31 über einen mit 30 bezeichneten Abschnitt bis zum Lager 4 spiralförmig um den Stössel herumgewickelt angeordnet. Von dort aus verlaufen die Leitungen durch eine der Luftausgleichsöffnungen 11 im Lager 3 bis zu den Steckkupplungen 12, 13. Die sich daran anschliessenden Leitungen 17, 18 führen über weiter durch entsprechende, nicht mehr dargestellte Kanäle zwischen Gehäuse 3 und Antriebsspule 8 in das Anschlusskabel 10.

Die Signalleitungen 24, 31 bestehen vorteilhafterweise aus isoliertem Litzendraht mit zwei verdrillten Einzeldrähten; alternativ kann auch ein dünnes Koaxialkabel verwendet werden.

Die spiralförmige Wicklung der Leitungen 24, 31 innerhalb des Leitungsabschnittes 30 erfolgt nach Durchführung der Leitung durch die Austrittsöffnung 28 und Einschrauben des Endteils 5 in den Stössel 2. Um die Montage zu erleichtern, kann an der Verbindungsstelle auch eine Steckverbindung vorgesehen sein.

Der Leitungsabschnitt 30 liegt im eingebauten Zustand an einem Anschlag 32 des Lagers 3 an und ist so weit zusammengedrückt, dass durch die Windungen eine Vorspannkraft $F_V$ gebildet wird, die der Reibungskraft $F_R$ des Stössels 2 entspricht und dieser bei der Vorwärtsbewegung des Stössels entgegenwirkt. Die Reibungskraft wird dabei weitgehend kompensiert. Der Vorspannweg $s_v$ der Feder ist dabei sehr viel grösser als der Stösselhub, so dass die Federkraft über den gesamten Stösselhub annähernd konstant bleibt.

Die Figur 5 zeigt eine Variante zu dem in Figur 2 gezeigten rückwärtigen Instrumententeil 1b, an der die Versorgungsleitung 10 angeschlossen ist. Die Magnetspule 8 ist im Gehäuseteil 1b teilweise axial vorstehend angeordnet und von einem Rückschlusselement 40 aus Weicheisenmaterial umgeben, welches mittels Isolierhülse 41 im Gehäuse 1b gehaltert ist. Der aus dem Gehäuseteil 1b vorstehende Teil der Isolierhülse 41 bildet so einen Steckzapfen für eine mechanische Verbindung mit dem hülsenförmigen Ende des Gehäuseteils 1a. Das Rückschlusselement 40 besteht aus zwei längs verlaufenden, durch geeignete Isoliermittel voneinander elektrisch isolierten Abschnitten 40a, 40b, die an ihren rückwärtigen Enden Anschlussglieder 42a, 42b für den Anschluss der zweiadrigen Signalleitung 17, 18 aufweisen und im gekuppelten Zustand die Leitungen 17, 18 des Instrumentenabschnittes 1a mit den Leitungen 24 und 31 des Instrumentenabschnittes 1b miteinander verbinden. Hierzu sind die Enden der Leitungen 24 und 31 mit federnden Kontaktzungen 43a, 43b versehen, welche beim axialen Aufschieben mit Gegenkontakten 44a, 44b der gegenüber dem vorderen Ende der Antriebsspule 8 vorstehenden Rückschlussegmente 40a, 40b im Sinne einer Kontaktgabe miteinander in Eingriff kommen.

Sind mehr als zwei Leitungen erforderlich, so sind entsprechend mehr solcher achsparallel verlaufender, gegeneinander elektrisch isolierter Abschnitte vorzusehen. Zur besseren Kontaktgabe ist die Kontaktfläche der Rückschlusselemente 40a, 40b zumindest im vorderen, überstehenden Bereich mit einem geeigneten Kontaktwerkstoff beschichtet, z.B. mit Nickel, Silber oder Platin.

Ein wesentlicher Vorteil der beschriebenen Anordnung ist, dass die elektrische Verbindung der Anschlussleitungen mit den Leitungen, die zum vorderen Instrumententeil führen, auf eine Weise gelöst ist, die gleichzeitig die magnetischen Eigenschaften der Antriebsspule verbessert. Dadurch, dass der zur Verbesserung der magnetischen Eigenschaften der Antriebsspule vorgesehene Eisenrückschlussring gleichsam zur elektrischen Kontaktierung mit benutzt wird, lässt sich neben den bereits erwähnten Vorteilen ein konstruktiv einfacherer Aufbau und eine erhöhte Betriebssicherheit erzielen.

Die Steuerung des Antriebs 7, 8 und damit der Geschwindigkeit des Stössels sowie die Signalauswertung aus dem Beschleunigungsaufnehmer 22 erfolgt wie in dem Patent 32 15 530 (deutsche Patentanmeldung DE-32 15 530) erläutert.

## Patentansprüche

1. Zahnärztliches Perkussionsinstrument mit einem als Handstück ausgebildeten Instrumentengehäuse (1), in dem ein an seinem einen Ende einen Prüfkopf (5) und an seinem anderen Ende einen Permanentmagneten enthaltender Stössel (2) axial beweglich gelagert ist sowie mit einer im Instrumentengehäuse (1) fest angeordneten Magnetspule (8), die zusammen mit dem Permanentmagneten (7) den Antrieb für den Stössel (2) bildet, dadurch gekennzeichnet, dass der Stössel (2) über seine gesamte Länge eine in Form und Abmessungen gleichbleibende Querschnittsfläche aufweist, dass der Permanentmagnet (7) im Inneren des Stössels (2) an dessen rückwärtiger Stirnseite angeordnet ist und dass die Magnetspule (8) in Verlängerung der Stösselachse hinter dem Stössel (2) angeordnet ist.

2. Perkussionsinstrument nach Anspruch 1, dadurch gekennzeichnet, dass das Instrumentengehäuse (1) in zwei lösbar miteinander verbundene Abschnitte (1a, 1b) unterteilt ist und die Magnetspule (8) mit dem einen, dem rückwärtigen Abschnitt (1b) eine nicht trennbare Einheit bildet.

3. Perkussionsinstrument nach Anspruch 2, dadurch gekennzeichnet, dass die Versorgungsleitung (10) und der rückwärtige Instrumentengehäuseabschnitt (1b) eine nicht trennbare Einheit bilden.

4. Perkussionsinstrument nach einem der Ansprüche 1 bis 3, dadurch gekennzeichnet, dass die Magnetspule (8) von einem Rückschlussring (40) umgeben ist, der aus mehreren achsparallel sich erstreckenden, elektrisch gegeneinander isolierten Abschnitten (40a, 40b) besteht, die zur Strom- und/oder Signalleitung herangezogen sind und zu diesem Zweck an ihren Enden Kontakte (42a, 42b, 44a, 44b) für den Anschluss der Leitungen (43, 24, 31) aufweisen oder bilden.

5. Perkussionsinstrument nach Anspruch 4, dadurch gekennzeichnet, dass der Rückschlussring (40) stirnseitig gegenüber dem einen Ende der Magnetspule (8) vorsteht und das vorstehende Ende Gegenkontakte (44a, 44b) für im anderen Instrumententeil (1a) angeordnete Kontaktglieder (43a, 43b) bildet.

6. Perkussionsinstrument nach Anspruch 5, dadurch gekennzeichnet, dass der Rückschlussring (40) von einer aus dem Instrumentengehäuseteil (1b) teilweise vorstehenden Isolierhülse (41) umgeben ist, deren vorstehender Teil einen Steckzapfen zur Verbindung der beiden Instrumententeile (1a, 1b) bildet.

7. Perkussionsinstrument nach einem der Ansprüche 4 bis 6, dadurch gekennzeichnet, dass die Oberfläche des Rückschlussringes (40) zumindest im Bereich der Kontaktierung mit einer Beschichtung aus Kontaktwerkstoff versehen ist.

8. Perkussionsinstrument nach einem der Ansprüche 1 bis 7, dadurch gekennzeichnet, dass der Stössel (2) in Gleitlagern (3, 4) gelagert ist, welche ein oder mehrere axiale Durchbrechungen (14) aufweisen.

9. Perkussionsinstrument nach Anspruch 8, dadurch gekennzeichnet, dass der Stössel (2) einen vorzugsweise zentrisch angeordneten und sich vom Prüfkopf (5) über einen wesentlichen Teil der Stössellänge erstreckenden Führungskanal (29) für eine Signalleitung (24, 31) eines im Prüfkopf (5) angeordneten Beschleunigungsaufnehmers (22) aufweist.

10. Perkussionsinstrument nach Anspruch 9, dadurch gekennzeichnet, dass die Signalleitung (24, 31) an einer radialen Öffnung (28) des Stössels (2) aus dem Führungskanal (29) herausgeführt ist und über einen bestimmten Längenabschnitt (30) spiralförmig um den Stössel (2) herumgewickelt ist.

11. Perkussionsinstrument nach Anspruch 9, dadurch gekennzeichnet, dass die Signalleitung (24, 31) Federeigenschaften besitzt, vorzugsweise ein isolierter Federdraht verwendet ist.

12. Perkussionsinstrument nach Anspruch 11, dadurch gekennzeichnet, dass als Signalleitung (24, 31) ein Litzendraht mit zwei verdrillten Einzeldrähten verwendet ist.

13. Perkussionsinstrument nach einem der Ansprüche 9 bis 12, dadurch gekennzeichnet, dass die Signalleitung (24, 31) über den Längenabschnitt (30), in dem sie spiralförmig um den Stössel (2) herumgewickelt ist, in der Ausgangsposition mit einer Vorspannkraft, die etwa der Reibungskraft des Stössels (2) entspricht, gegen einen Anschlag (32) anliegend eingespannt ist.

14. Perkussionsinstrument nach Anspruch 13, dadurch gekennzeichnet, dass der Vorspannweg, mit dem die Signalleitung (24, 31) in diesem Längenabschnitt (30) eingespannt ist, sehr viel grösser ist als der maximale Stösselhub.

**Revendications**

1. Instrument dentaire à percussion comportant un boîtier (1), qui est réalisé sous la forme d'une pièce à main et dans lequel un coulisseau (2) contenant, à l'une de ses extrémité, une tête de sondage (5) et, à son autre extrémité, un aimant permanent, est monté de manière à être déplaçable axialement, et comportant une bobine magnétique (8) montée fixe dans le boîtier (1) de l'instrument et formant, conjointement avec l'aimant permanent (4), le dispositif d'entraînement pour le coulisseau (2), caractérisé par le fait que le coulisseau (2) possède, sur toute sa longueur, une surface en coupe transversale dont la forme et les dimensions restent constantes, que l'aimant permanent (7) est disposé à l'intérieur du coulisseau (2), sur la face frontale arrière de ce dernier et que la bobine magnétique (8) est disposée dans le prolongement de l'axe du coulisseau (2), en arrière de ce dernier.

2. Instrument à percussion suivant la revendication 1, caractérisé par le fait que le boîtier (1) de l'instrument est utilisé en deux sections (1a, 1b) reliées entre elles de façon détachable et que la bobine magnétique (8) forme, avec une première section, à savoir la section arrière (1b), une unité indivisible.

3. Instrument à percussion suivant la revendication 2, caractérisé par le fait que la canalisation d'alimentation (10) et la section arrière (1b) du boîtier de l'instrument forment une unité indivisible.

4. Instrument à percussion suivant l'une des revendications 1 à 3, caractérisé par le fait que la bobine magnétique (8) est entourée par un anneau de fermeture du flux (40), qui est constitué par plusieurs sections (40a, 40b) qui s'étendent parallèlement à l'axe, sont isolées électriquement les unes par rapport aux autres, sont utilisées pour véhiculer le courant et/ou des signaux et possèdent ou forment à cet effet, au niveau de leurs extrémités, des contacts 42a, 42b; 44a, 44b) pour le raccordement des conducteurs (43, 24, 31).

5. Instrument à percussion suivant la revendication 4, caractérisé par le fait que l'anneau de fermeture du flux (40) fait saillie frontalement par rapport à la première extrémité de la bobine magnétique (8) et que l'extrémité saillante forme des contacts antagonistes (44a, 44b) pour des organes de contact (43a, 43b) disposés dans l'autre partie de l'instrument.

6. Instrument à percussion suivant la revendication 5, caractérisé par le fait que l'anneau de fermeture du flux (40) est entouré par une douille isolante (40), qui fait saillie partiellement hors de la partie (1b) du boîtier de l'instrument et dont la partie saillante forme un embout d'enfichage servant à relier les deux parties (1a, 1b) de l'instrument.

7. Instrument à percussion suivant l'une des revendications 4 à 6, caractérisé par le fait que la surface de l'anneau de fermeture du flux (40) comporte, au moins dans la zone d'établissement des contacts, un revêtement formé d'un matériau de contact.

8. Instrument à percussion suivant l'une des revendications 1 à 7, caractérisé par le fait que le coulisseau (2) est monté dans les paliers lisses (3, 4), qui possèdent un ou plusieurs perçages axiaux (14).

9. Instrument à percussion suivant la revendication 8, caractérisé par le fait que le coulisseau (2) possède un canal de guidage (29), qui, de préférence, est centré et s'étend à partir de la tête de sondage (5) sur une partie importante de la longueur du coulisseau, pour un conducteur (24, 31) de transmission de signaux d'un capteur d'accélération (22) disposé dans la tête de contrôle (5).

10. Instrument à percussion suivant la revendication 9, caractérisé par le fait que le conducteur (24, 31) de transmission de signaux est ressorti du canal de guidage (29) au niveau d'une ouverture radiale (28) du coulisseau (2), et est enroulé sous une forme hélicoïdale autour du poussoir (2), sur une section de longueur déterminée (30).

11. Instrument à percussion suivant la revendication 9, caractérisé par le fait que le conducteur (24, 31) de transmission de signaux possède des caractéristiques d'un ressort et qu'on utilise de préférence un fil isolé pour ressort.

12. Instrument à percussion suivant la revendication 11, caractérisé par le fait qu'on utilise comme conducteur (24, 31) de transmission de signaux, un fil torsadé formé de deux fils individuels torsadés.

13. Instrument à percussion suivant l'une des revendications 9 à 12, caractérisé par le fait que dans la position initiale, le conducteur (24, 31) de

9 EP 0 184 746 B1 10

transmission de signaux est serré en étant appliqué contre une butée (32), sous l'action d'une force de serrage correspondant approximativement à la force de frottement du coulisseau (2), sur la section de longueur (30), sur laquelle il est enroulé en hélice autour du coulisseau (2).

14. Instrument à percussion suivant la revendication 13, caractérisé par le fait que la course de précontrainte, sur laquelle le conducteur (24, 31) de transmission de signaux est serré dans cette section de longueur (3), est très supérieure à la course maximale du coulisseau.

**Claims**

1. Dental percussion instrument with an instrument casing (1) constructed as a hand-piece, in which a striker (2) is mounted in an axially movable manner with a testing head (5) at one end and a permanent magnet at the other end, and with a magnetic coil (8) arranged fixedly in the instrument casing (1), the magnetic coil, together with the permanent magnet (7), forming the drive for the striker (2), characterised in that the striker (2) has over its entire length a cross-sectional area whose form and measurements remain constant, in that the permanent magnet (7) is arranged inside the striker (2) on its rear end face, and in that the magnetic coil (8) is arranged behind the striker (2) in a prolongation of the striker axis.

2. Percussion instrument according to claim 1, characterised in that the instrument casing (1) is subdivided into two sections (1a, 1b), which are detachably connected together, and the magnetic coil (8) forms a inseparable unit with one of the sections, the rearward one (1b).

3. Percussion instrument according to claim 2, characterised in that the supply lead (10) and the rearward section (1b) of the instrument casing form an inseparable unit.

4. Percussion instrument according to one of claims 1 to 3, characterised in that the magnetic coil (8) is surrounded by a return ring (40), which comprises several sections (40a, 40b) which extend axially parallel and are electrically insulated from each other, wherein these sections are utilised for currend and/or signal conduction and for this purpose have or form contacts (42a, 42b; 44a, 44b) at their ends in order to connect the leads (43, 24, 31).

5. Percussion instrument according to claim 4, characterised in that the return ring (40) projects at its end with respect to one of the ends of the magnetic coil (8), and the projecting end forms counter-contacts (44a, 44b) for contact elements (43a, 43b) arranged in the other part of the instrument (1a).

6. Percussion instrument according to claim 5, characterised in that the return ring (40) is surrounded by an insulating casing (41) which projects partially from the part (1b) of the instrument case, the projecting part of the insulating casing forming a centre spigot in order to connect the two parts (1a, 1b) of the instrument.

7. Percussion instrument according to one of claims 4 to 6, characterised in that the surface of the return ring (40) is provided with a coating of contact material in at least the region of contact.

8. Percussion instrument according to one of claims 1 to 7, characterised in that the striker (2) is stored in slide bearings (3, 4), which have one or more axial openings (14).

9. Percussion instrument according to claim 8, characterised in that the striker (2) has a guide channel (29) for one signal lead (24, 31) of an acceleration detector (22) arranged in the testing head (5), wherein this guide channel is arranged preferably centrally and extends from the testing head (5) over a substantial part of the striker length.

10. Percussion instrument according to claim 9, characterised in that the signal lead (24, 31) is conducted out of the guide channel (29) at a radial opening (28) of the striker (2) and is wound spirally around the striker (2) along a specified longitudinal section (30).

11. Percussion instrument according to claim 9, characterised in that the signal lead (24, 31) has the properties of a spring, an insulated spring wire being preferably used.

12. Percussion instrument according to claim 11, characterised in that a stranded wire with two twisted individual wires is used as a signal lead.

13. Percussion instrument according to one of claims 9 to 12, characterised in that the signal lead (24, 31) along the longitudinal section (30) in which the signal lead (24, 31) is wound spirally around the striker (2) is clamped in the starting position resting against a stop (32) with a biasing force which approximately corresponds to the frictional force of the striker (2).

14. Percussion instrument according to claim 13, characterised in that the path over which the signal lead (24, 31) in this longitudinal section is spring-biased is much greater than the maximal striker stroke.

1/1

FIG 1

FIG 2

FIG 3

FIG 4

FIG 5

FIG 6